# EUROPEAN PATENT SPECIFICATION

(11) **EP 2 735 358 B1**
(45) Date of publication and mention of the grant of the patent: **10.01.2018**
(21) Application number: 12193791.6
(22) Date of filing: 22.11.2012
(51) Int. Cl.: B01D 63/02, A61M 1/16, B01D 65/00, F28F 21/06

(54) **Capillary dialyzers**
Hohlfaserdialysatoren
Dialyseurs capillaires

(43) Date of publication of application: 28.05.2014
(73) Proprietor: Gambro Lundia AB, 220 10 Lund (SE)
(72) Inventor: Wagner, Steffen, 72469 Meßstetten (DE); Beck, Christof, 72475 Bitz (DE); Blickle, Rainer, 72475 Bitz (DE); Ermantraut, Stefan, 72336 Balingen (DE); Hertzler, Bernd, 72336 Balingen (DE)
(74) Representative: Perchenek, Nils

(56) References cited:
- EP-A1- 1 398 047
- EP-A2- 1 864 709
- US-A- 4 038 190
- US-A- 4 343 668
- US-A1- 2003 010 718
- US-A1- 2003 102 264

## Description

### Technical Field

The present invention relates to capillary dialyzers for blood purification.

### Background of the Invention

Capillary dialyzers are widely used for blood purification in patients suffering from renal insufficiency, i.e., for treatment of the patients by hemodialysis, hemodiafiltration or hemofiltration. A multitude of different models of capillary dialyzers is commercially available.

The devices generally consist of a casing comprising a tubular section with end caps capping the mouths of the tubular section. A bundle of hollow fiber membranes is arranged in the casing in a way that a seal is provided between the first flow space formed by the fiber cavities and a second flow space surrounding the membranes on the outside. Examples of such devices are disclosed in EP 0 844 015 A2, EP 0 305 687 A1, and WO 01/60477 A2.

US 4 038 190 discloses a hollow fiber module for dialysis with a casing which has a longitudinal U shaped bulge/channel which is opened at the top as well as at the short sides. On the top a lid is provided and at the short sides end caps.

EP 1 864 709 is a membrane contactor for e.g. filtering liquids. It has a hollow casing with only one separate end cap. The hollow fibers extend from the end cap into the hollow body.

In general commercially available dialyzers at least comprise one housing and two end caps. Generally, they comprise additional parts like sealing rings, gaskets, and support rings. Production, handling, and subsequent assembly of the individual elements add to the complexity of the production process and the manufacturing cost. It would therefore be desirable to reduce the number of individual parts in the dialyzer.

### Summary of the Invention

A capillary dialyzer is provided which does not comprise end caps, sealing rings, or support rings.

This invention provides for a capillary dialyzer according to claim 1.

This invention also provides for a process for producing a capillary dialyzer according to claim 8.

This disclosure also describes a dialyzer shell which comprises a single trough-shaped molded part 1 having blood ports 3 and dialysate ports 4; and a cover 2 bonded to the seam of the trough-shaped molded part and sealing the opening of the trough.

### Short Description of the Drawings

- Fig. 1: shows a perspective view of a capillary dialyzer of the present application;
- Fig. 2: shows a partially exploded perspective view of a capillary dialyzer of the present application;
- Fig. 3: shows a perspective view of an embodiment of the housing of the capillary dialyzer of the present application;
- Fig. 4: shows a perspective view and a cross-sectional view of a bundle of hollow fibers in an embodiment of the housing of the capillary dialyzers of the present invention and a casting mold used during potting of the ends of the bundle of hollow fibers;
- Fig. 5: shows a detail of a cross-sectional top view and a detail of a longitudinal-sectional perspective view of a bundle of hollow fibers in an embodiment of the housing of the capillary dialyzers of the present invention after potting of the ends of the hollow fibers and before cutting open the ends of the hollow fibers.

### Detailed Description

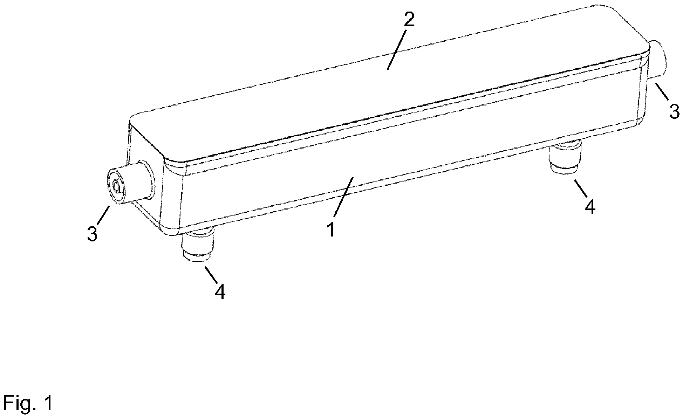

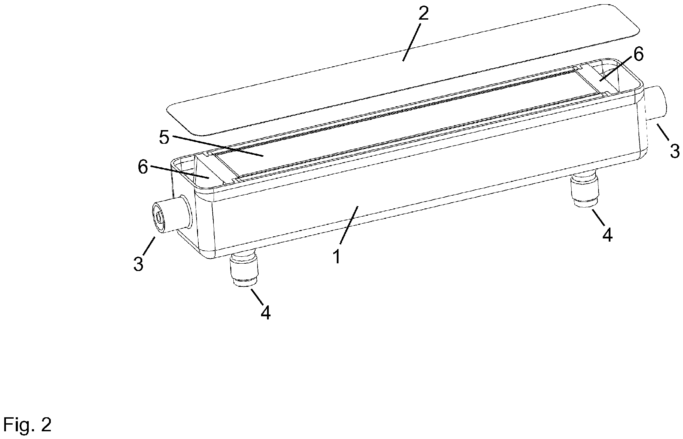

Figs. 1 and 2 show a capillary dialyzer comprising:
a) a trough-shaped housing **1** defining a longitudinally extending internal chamber including a first end and a second end;
b) a cover **2** sealing the opening of the trough of the housing **1;**
c) a bundle **5** of semi-permeable hollow fiber membranes disposed within the internal chamber and extending longitudinally from the first end of the housing to the second end of the housing, the hollow fiber membranes having an outer surface, and a first end and a second end corresponding to the first end and the second end of the internal chamber;
d) end wall means **6** supporting the first and second ends of the hollow fiber membranes within the internal chamber so as to sealingly separate the first and second ends of the hollow fiber membranes from the outer surface of the hollow fiber membranes between the first and second ends thereof;
e) blood ports **3** on the front end and rear end, respectively, of the housing **1;** and dialysis fluid ports **4** on the wall of the housing at locations between the first and second end of the bundle **5** of hollow fiber membranes.

The housing **1** of the capillary dialyzers of the present invention is a trough-shaped molded part which includes the functionality provided by the end caps in conventional dialyzers. It can be produced in a single step by injection molding of a suitable thermoplastic polymer. Suitable polymers include thermoplastic polymers, e.g., polyethylene, polypropylene, polyesters like PET or PBT, polymethylmethacrylate, polystyrene (HIPS), thermoplastic polyurethane, or polycarbonate. In one embodiment of the present invention, the housing **1** is transparent. In another embodiment, the housing **1** is opaque.

The housing **1** comprises two blood ports **3** on its front end and rear end, respectively. During operation of the dialyzer, the blood ports **3** are used to feed blood to the dialyzer and remove blood from the dialyzer, respectively. Two dialysis fluid ports **4** are provided on the wall of the housing at locations between the first and second end of the bundle **5** of hollow fiber membranes. In one embodiment of the invention shown in Fig. 1, both dialysis fluid ports **4** are located on the bottom side of the housing, near the end wall means **6.** In another embodiment of the invention, at least one of the dialysis ports **4** is located on a side wall of the housing **1.** In still another embodiment of the invention, both dialysis fluid ports **4** are located on the same side wall of the housing **1.** In still another embodiment of the invention, one port is located on one of the side walls and the other port is located on the opposite side wall of the housing **1.**

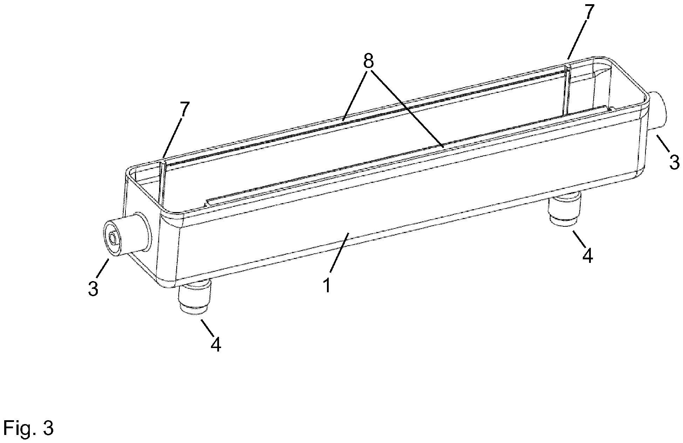

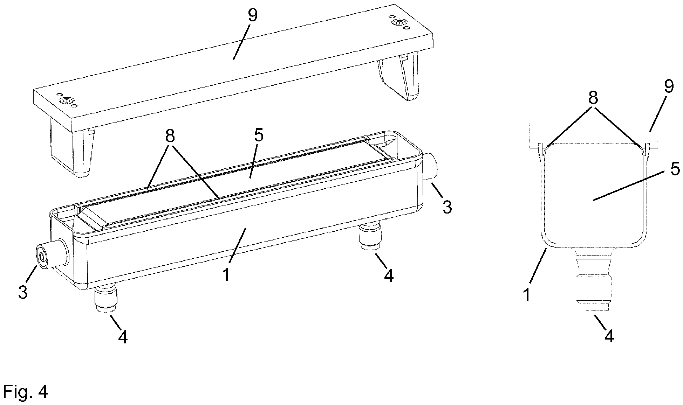

In one embodiment of the invention, the housing **1** additionally comprises flexible ears **8** located at the upper part of the inside of each side wall of the housing **1** near the trough opening and extending longitudinally along the housing **1,** as shown in Figs. 3 and 4. The flexible ears **8** facilitate introduction of the bundle **5** of hollow fiber membranes into the housing **1** by providing additional guidance and act as an additional retainer for the bundle **5** of hollow fiber membranes when bent into their final position, as shown in Fig. 4.

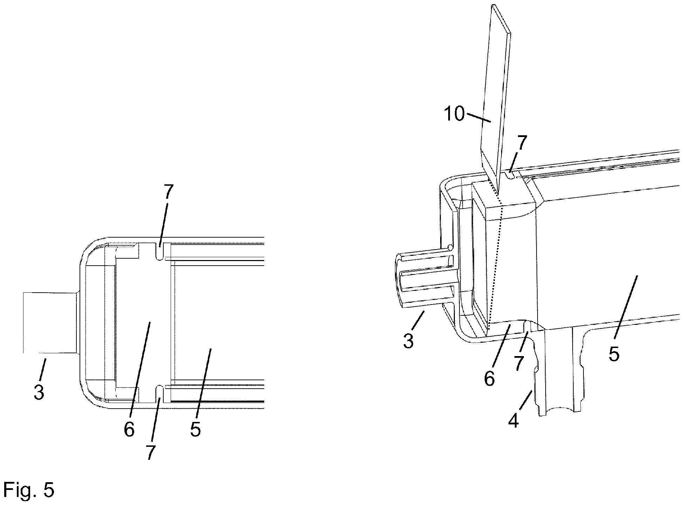

In another embodiment of the invention, as shown in Figs. 3 and 5, the housing **1** also comprises circumferential ridges **7** in the part of the housing **1** where the end wall means **6** are located. The circumferential ridges **7** serve as a support for the bundle **5** of hollow fiber membranes and a spacer between the ends of the hollow fiber membranes and the inner wall of the housing **1** before the potting step. After completion of the potting step, the circumferential ridges **7** provide an additional anchor for the end wall means **6.** In one embodiment, the circumferential ridges **7** have an elevation, relative to the inner wall surface of the housing (**1**), in the range of from 1 to 10 mm, for instance, 2 to 5 mm.

The cover **2** seals the opening of the trough of the housing **1.** The cover **2** is a sheet of a transparent polymer. Suitable transparent polymers include polyethylene, polypropylene, polyurethane, and polycarbonate. The thickness of the sheet generally is in the range of from 0.1 to 0.5 mm, for instance, 0.15 to 0.3 mm. The cover **2** is bonded to the seam of the trough-shaped molded part, thus sealing the opening of the trough. The cover **2** is bonded to the housing **1** and the end wall means **6** by a suitable process, e.g., welding, heat-sealing, or by means of an adhesive. The cover **2** can be equipped with further functions. In one embodiment of the dialyzer, a strain gauge is provided on the cover **2,** allowing for detection of strain due to the internal pressure in the dialyzer and thus, measurement of the pressure within the dialyzer. In one embodiment, the calibration curve of the strain gauge is recorded during the integrity test of the dialyzer and printed on the cover as a Data Matrix code. When the dialyzer is connected to a dialysis monitor, the Data Matrix code can be read by the monitor and used to calculate the pressure within the dialyzer from the observed distortion of the strain gauge.

The end wall means **6** supporting the first and second ends of the hollow fiber membranes within the internal chamber are generated by potting the ends of the fiber with a reactive polymer. A suitable potting material for the hollow fiber membranes is polyurethane.

The present disclosure also relates to processes for producing the dialyzer of the invention according to claim 8. A process also described herein comprises the steps of
i) providing a trough-shaped housing **1** defining a longitudinally extending internal chamber including a first end and a second end; and having blood ports **3** on its front end and rear end wall, respectively; and dialysis fluid ports **4** on its bottom wall and/or side walls;
ii) introducing a bundle **5** of hollow fiber membranes into the internal chamber of the housing **1,** so that the fibers extend longitudinally from the first end of the housing **1** to the second end of the housing **1;**
iii) introducing a casting mold **9** into the housing **1** which completely covers the trough opening of the housing **1** and seals off compartments at the first end and at the second end, respectively, of the interior chamber;
iv) potting the ends of the hollow fiber membranes to produce end wall means **6** supporting the ends of the hollow fiber membranes within the internal chamber;
v) removing the casting mold **9** from the housing **1;**
vi) cutting the part of the end wall means **6** facing the blood ports **3** to open the ends of the hollow fiber membranes; and
vii) bonding the cover **2** to the trough opening of the housing **1.**

It is expedient to close the ends of the hollow fiber membranes before the potting step in order to prevent the potting material from permeating into the fibers. The fiber ends can be closed by processes known in the art, e.g., by melting or by means of an adhesive. In one embodiment of the process the ends of the hollow fiber membranes are closed before the bundle **5** of hollow fiber membranes is introduced into the housing **1.**

After the bundle **5** of hollow fiber membranes has been introduced into the housing **1,** a casting mold **9** is introduced into the housing **1.** As shown in Fig. 4 for an exemplary embodiment, the casting mold **9** in its final position completely covers the trough opening of the housing **1** and seals off compartments at the first end and at the second end, respectively, of the interior chamber, so that potting material introduced through one or both of the dialysis ports **4** cannot penetrate into these compartments.

In one embodiment of the process, the casting mold **9** also provides spacers between a part of the end wall means **6** on the side facing the blood port **3** and the side walls of the housing **1,** as shown in Fig. 4. As a result, gaps are provided between a portion of the end wall means **6** and the side walls of the housing **1,** as shown in Fig. 5.

In an embodiment of the invention where the housing **1** additionally comprises flexible ears **8,** the casting mold **9** also bends the flexible ears **8** into their final position during its introduction into the housing **1,** simultaneously pushing peripheral hollow fiber membranes into the housing **1** and further compacting the bundle **5** of hollow fiber membranes, as illustrated by Fig. 4.

With the casting mold **9** in its final position, the ends of the hollow fiber membranes are potted to produce end wall means **6.** Potting material, for instance polyurethane, is introduced into the housing via one or both of the dialysis fluid ports **4.** The potting material is distributed within the housing **1** of the dialyzer by centrifugation, i.e., rotating the dialyzer at high speed perpendicular to its longitudinal axis. The potting material is allowed to cure; thereby forming end wall means **6** on both ends of the bundle **5** of hollow fiber membranes. After the potting material has hardened, the casting mold **9** is removed.

The ends of the hollow fiber membranes are subsequently opened, for instance by cutting the part of the end wall means **6** facing the blood ports **3** with a blade **10.** The end wall means **6** are cut diagonally, as shown in Fig. 5. In one embodiment of the process, the angle and the starting position of the blade **10** are selected to have the blade **10** cut through the face of the end wall means **6** at a position above the bottom wall of the housing **1,** e.g., at a distance in the range of from 0.5 to 2 mm. The cutting angle, relative to a plane perpendicular to the bottom wall of the housing **1,** is in the range of from 2 to 10°. As the resulting wedge-shaped section does not adhere to the bottom wall of the housing **1,** it can easily be removed from the housing **1.** This is even more so for embodiments where a gap is provided between the section to be cut off from the end wall means **6** and the side walls of the housing **1,** as shown in Fig. 5. In one embodiment of the process, the cuts at both end wall means **6** of the dialyzer are mirror-symmetrical to each other.

After the ends of the hollow fiber membranes have been opened, the cover **2** is mounted on the housing **1** of the dialyzer to close the trough opening of the housing **1.** The cover **2** is bonded to the housing **1** and the end wall means **6** by a suitable process, e.g., welding, heat-sealing, or by means of an adhesive, for instance, a polyurethane adhesive.

## Claims

1. A capillary dialyzer comprising:
a) a trough-shaped housing (**1**) defining a longitudinally extending internal chamber including a first end and a second end;
b) a cover (**2**) sealing the opening of the trough of the housing (**1**), the cover (**2**) being a sheet of a transparent polymer;
c) a bundle (**5**) of semi-permeable hollow fiber membranes disposed within the internal chamber and extending longitudinally from the first end of the housing to the second end of the housing, the hollow fiber membranes having an outer surface, and a first end and a second end corresponding to the first end and the second end, respectively, of the internal chamber;
d) end wall means (**6**) supporting the first and second ends of the hollow fiber membranes within the internal chamber so as to sealingly separate the first and second ends of the hollow fiber membranes from the outer surface of the hollow fiber membranes between the first and second ends thereof;
e) blood ports (**3**) on the front end and rear end, respectively, of the housing (**1**); and dialysis fluid ports (**4**) on the wall of the housing at locations between the first and second end of the bundle (**5**) of hollow fiber membranes.

2. The capillary dialyzer of claim 1, wherein the housing (**1**) further comprises circumferential ridges (**7**) within the end wall means (**6**).

3. The capillary dialyzer of claim 2, wherein the circumferential ridges (**7**) have an elevation relative to the inner wall surface of the housing (**1**) which is in the range of from 1 to 10 mm.

4. The capillary dialyzer of any one of claims 1 to 3, wherein the transparent polymer of the cover (**2**) is polypropylene.

5. The capillary dialyzer of any one of claims 1 to 4, wherein a strain gauge is provided on the cover (**2**).

6. The capillary dialyzer of any one of claims 1 to 5, wherein the trough-shaped molded part (**1**) is comprised of polypropylene.

7. The capillary dialyzer of any one of claims 1 to 5, wherein the trough-shaped molded part (**1**) is comprised of polycarbonate.

8. A process for producing a capillary dialyzer comprising the steps of
i) providing a trough-shaped housing (**1**) defining a longitudinally extending internal chamber including a first end and a second end; and having blood ports (**3**) on its front end wall and rear end wall, respectively; and dialysis fluid ports (**4**) on its bottom wall and/or side walls;
ii) introducing a bundle (**5**) of hollow fiber membranes into the internal chamber of the housing (**1**), so that the fibers extend longitudinally from the first end of the housing (**1**) to the second end of the housing (**1**) ;
iii) introducing a casting mold (**9**) into the housing (**1**) which completely covers the trough opening of the housing (**1**) and seals off compartments at the first end and at the second end, respectively, of the interior chamber;
iv) potting the ends of the hollow fiber membranes to produce end wall means (**6**) supporting the ends of the hollow fiber membranes within the internal chamber;
v) removing the casting mold (**9**) from the housing (**1**) ;
vi) cutting the part of the end wall means (**6**) facing the blood ports (**3**) to open the ends of the hollow fiber membranes; and
vii) bonding a cover (**2**) to the trough opening of the housing (**1**).

9. The process of claim 8, wherein the casting mold (**9**) is shaped to provide spacers between a part of the end wall means (**6**) on the side facing the blood port (**3**) and the side walls of the housing (**1**).

10. The process of claims 8 or 9, wherein the end wall means (**6**) are cut with a blade (**10**) at an angle, relative to a plane perpendicular to the bottom wall of the housing (**1**), in the range of from 2 to 10°.

## Patentansprüche

1. Kapillardialysator umfassend:
a) ein trogförmiges Gehäuse (**1**), welches eine sich in Längsrichtung erstreckende interne Kammer mit einem ersten und einem zweiten Ende definiert;
b) eine Abdeckung (**2**), welche die Öffnung des Trogs des Gehäuses (**1**) verschließt, wobei die Abdeckung (**2**) eine Folie aus transparentem Polymer ist;
c) ein Bündel (**5**) semi-permeabler Hohlfasermembranen, welches in der internen Kammer angeordnet ist und sich in Längsrichtung von dem ersten Ende des Gehäuses zu dem zweiten Ende des Gehäuses erstreckt, wobei die Hohlfasermembranen eine äußere Oberfläche und ein erstes und ein zweites Ende aufweisen, die dem ersten Ende bzw. dem zweiten Ende der internen Kammer entsprechen;
d) Endwandmittel (**6**), welche die ersten und zweiten Enden der Hohlfasermembranen so innerhalb der internen Kammer aufnehmen, dass die ersten und zweiten Enden der Hohlfasermembranen dichtend von der äußeren Oberfläche der Hohlfasermembranen zwischen deren ersten und zweiten Enden abgeschottet sind;
e) Blutanschlüsse (**3**) am vorderen Ende und hinteren Ende des Gehäuses (**1**); und Dialyseflüssigkeitsanschlüsse (**4**) an der Wand des Gehäuses an Positionen zwischen dem ersten und zweiten Ende des Bündels(**5**) von Hohlfasermembranen.

2. Kapillardialysator gemäß Anspruch 1, worin das Gehäuse (**1**) außerdem umlaufende Rippen (**7**) innerhalb der Endwandmittel (**6**) aufweist.

3. Kapillardialysator gemäß Anspruch 2, worin die umlaufenden Rippen (**7**) eine Höhe im Bereich von 1 bis 10 mm aufweisen, bezogen auf die Oberfläche der Innenwand des Gehäuses (**1**).

4. Kapillardialysator gemäß einem der Ansprüche 1 bis 3, worin das transparente Polymer der Abdeckung (**2**) Polypropylen ist.

5. Kapillardialysator gemäß einem der Ansprüche 1 bis 4, worin auf der Abdeckung (**2**) ein Dehnungsmessstreifen angeordnet ist.

6. Kapillardialysator gemäß einem der Ansprüche 1 bis 5, worin das trogförmige Formteil (**1**) aus Polypropylen besteht.

7. Kapillardialysator gemäß einem der Ansprüche 1 bis 5, worin das trogförmige Formteil (**1**) aus Polycarbonat besteht.

8. Verfahren zur Herstellung eines Kapillardialysators, umfassend die Schritte:
i) Bereitstellen eines trogförmigen Gehäuses (**1**), welches eine sich in Längsrichtung erstreckende interne Kammer mit einem ersten und einem zweiten Ende definiert; und Blutanschlüsse (**3**) an den Wänden seines vorderen und hinteren Endes; und Dialyseflüssigkeitsanschlüsse (**4**) an seiner Bodenplatte und/oder seinen Seitenwänden aufweist;
ii) Einbringen eines Bündels (**5**) von Hohlfasermembranen in die interne Kammer des Gehäuses (**1**), so dass die Fasern sich in Längsrichtung vom ersten Ende des Gehäuses (**1**) zum zweiten Ende des Gehäuses (**1**) erstrecken;
iii) Einbringen einer Gießform (**9**) in das Gehäuse (**1**), welche die Trogöffnung des Gehäuses (**1**) vollständig abdeckt und Räume am ersten und zweiten Ende der internen Kammer abschottet;
iv) Eingießen der Enden der Hohlfasermembranen um Endwandmittel (**6**) zu erzeugen, welche die Enden der Hohlfasermembranen innerhalb der internen Kammer aufnehmen;
v) Entfernen der Gießform (**9**) aus dem Gehäuse (**1**);
vi) Abschneiden des den Blutanschlüssen (**3**) zugewandten Teils der Endwandmittel (**6**), um die Enden der Hohlfasermembranen zu öffnen; und
vii) Aufsiegeln einer Abdeckung (**2**) auf die Trogöffnung des Gehäuses (**1**).

9. Verfahren gemäß Anspruch 8, worin die Gießform (**9**) so geformt ist, dass sie Abstandshalter zwischen einem Teil der Endwandmittel (**6**) auf der den Blutanschlüssen (**3**) zugewandten Seite und den Seitenwänden des Gehäuses (**1**) bereitstellt.

10. Verfahren gemäß Anspruch 8 oder 9, worin die Endwandmittel (**6**) in einem Winkel im Bereich von 2 bis 10°, bezogen auf eine zur Bodenplatte des Gehäuses (**1**) lotrechte Fläche, mit einer Klinge (**10**) geschnitten werden.

## Revendications

1. Dialyseur capillaire comprenant :
a) un logement en forme d'auge (**1**) définissant une chambre interne s'étendant longitudinalement comprenant une première extrémité et une seconde extrémité ;
b) un couvercle (**2**) fermant hermétiquement l'ouverture de l'auge du logement (**1**), le couvercle (**2**) étant une feuille de polymère transparent ;
c) un faisceau (**5**) de membranes à fibres creuses semi-perméables disposé à l'intérieur de la chambre interne et s'étendant longitudinalement depuis la première extrémité du logement jusqu'à la seconde extrémité du logement, les membranes à fibres creuses ayant une surface externe, et une première extrémité et une seconde extrémité correspondant à la première extrémité et à la seconde extrémité, respectivement, de la chambre interne ;
d) des moyens de paroi d'extrémité (**6**) supportant les première et seconde extrémités des membranes à fibres creuses à l'intérieur de la chambre interne de façon à séparer de façon hermétique les première et seconde extrémités des membranes à fibres creuses de la surface externe des membranes à fibres creuses entre leurs première et seconde extrémités ;
e) des orifices (**3**) pour le sang sur l'extrémité avant et l'extrémité arrière, respectivement, du logement (**1**) ; et des orifices (**4**) pour le fluide de dialyse sur la paroi du logement au niveau d'emplacements compris entre la première et la seconde extrémité du faisceau (**5**) de membranes à fibres creuses.

2. Dialyseur capillaire selon la revendication 1, dans lequel le logement (**1**) comprend en outre des nervures circonférentielles (**7**) à l'intérieur des moyens de paroi d'extrémité (**6**).

3. Dialyseur capillaire selon la revendication 2, dans lequel les nervures circonférentielles (**7**) ont une élévation par rapport à la surface de paroi interne du logement (**1**) qui est dans la plage de 1 à 10 mm.

4. Dialyseur capillaire selon l'une quelconque des revendications 1 à 3, dans lequel le polymère transparent du couvercle (**2**) est du polypropylène.

5. Dialyseur capillaire selon l'une quelconque des revendications 1 à 4, dans lequel une jauge de contrainte est prévue sur le couvercle (**2**).

6. Dialyseur capillaire selon l'une quelconque des revendications 1 à 5, dans lequel la partie moulée en forme d'auge (**1**) est constituée de polypropylène.

7. Dialyseur capillaire selon l'une quelconque des revendications 1 à 5, dans lequel la partie moulée en forme d'auge (**1**) est constituée de polycarbonate.

8. Procédé de production d'un dialyseur capillaire comprenant les étapes consistant à
i) fournir un logement en forme d'auge (**1**) définissant une chambre interne s'étendant longitudinalement comprenant une première extrémité et une seconde extrémité ; et ayant des orifices (**3**) pour le sang sur sa paroi d'extrémité avant et sa paroi d'extrémité arrière, respectivement ; et des orifices (**4**) pour le fluide de dialyse sur sa paroi inférieure et/ou ses parois latérales ;
ii) introduire un faisceau (**5**) de membranes à fibres creuses dans la chambre interne du logement (**1**), de sorte que les fibres s'étendent longitudinalement depuis la première extrémité du logement (**1**) jusqu'à la seconde extrémité du logement (**1**) ;
iii) introduire un moule de coulée (**9**) dans le logement (**1**) qui recouvre entièrement l'ouverture de l'auge du logement (**1**) et qui isole hermétiquement des compartiments au niveau de la première extrémité et de la seconde extrémité, respectivement, de la chambre intérieure ;
iv) enrober les extrémités des membranes à fibres creuses pour produire des moyens de paroi d'extrémité (**6**) supportant les extrémités des membranes à fibres creuses à l'intérieur de la chambre interne ;
v) retirer le moule de coulée (**9**) du logement (**1**) ;
vi) découper la partie des moyens de paroi d'extrémité (**6**) tournée vers les orifices (**3**) pour le sang pour ouvrir les extrémités des membranes à fibres creuses ; et
vii) lier un couvercle (**2**) à l'ouverture de l'auge du logement (**1**).

9. Procédé selon la revendication 8, dans lequel le moule de coulée (**9**) est façonné pour fournir des espaceurs entre une partie des moyens de paroi d'extrémité (**6**) sur le côté tourné vers l'orifice (**3**) pour le sang et les parois latérales du logement (**1**).

10. Procédé selon les revendications 8 ou 9, dans lequel les moyens de paroi d'extrémité (**6**) sont découpés avec une lame (**10**) selon un angle, par rapport à un plan perpendiculaire à la paroi inférieure du logement (**1**), dans la plage de 2 à 10°.
